# EUROPEAN PATENT APPLICATION

(11) **EP 3 321 574 A1**
(43) Date of publication of application: **16.05.2018**
(21) Application number: 16198568.4
(22) Date of filing: 14.11.2016
(51) Int. Cl.: F21V 9/08, A61N 5/06, F21V 29/67, F21W 131/20

(54) **ILLUMINATION DEVICE**

(71) Applicant: Koninklijke Philips N.V., 5656 AE Eindhoven (NL)
(72) Inventor: BABBAGE, Sean Joel, 5656 AE Eindhoven (NL); SCHOTT, René Heinrich Augustinus, 5656 AE Eindhoven (NL)
(74) Representative: de Haan, Poul Erik

(57) **Abstract**

Illumination device (1) comprising a housing (2) having first (S1) and second oppsite sides (S2), at least one inlet (14) at the first side (S1), at least one outlet (15) at the second side (S2), an illumination source (3) in the housing (2), a reflector (8) which at least partially surrounds the illumination source (3), a fan (5) arranged on the first side (S1) fluidly connected to the least one inlet (14), and a plate (4) on the second side (S2), wherein the at least one outlet (15) is arranged at an outer circumference of the plate (4), and wherein the fan (5) is configured to draw cold ambient air (C) into the housing (2) through the at least one inlet (14), guide the cold ambient air (C) around an outer surface of the reflector (8) and discharge heated air (H) through the at least one outlet (15).

## Description

### FIELD OF THE INVENTION

The present invention relates to an illumination device with an illumination source arranged in a housing. A fan draws in cold outside air and directs the air flow along predetermined flow-paths. The illumination source might emit light in different wavelength ranges, especially in the infrared region of the spectrum and is suitable for medical and health-care purposes.

### BACKGROUND OF THE INVENTION

State of the art infrared lamps typically use a halogen or incandescent light source and a glass filter to remove unwanted wavelengths, thus reducing the output to infrared only. These lamps usually are used in the field of health care for example to relieve pain of muscles and offer relaxation.

As these lamps are typically of higher power (100-650 Watts), the outside surface of the lamp typically becomes quite hot as well, because a halogen or incandescent bulb as infrared light source is generating also a lot of heat. The heat is a necessary part of the therapy, but only in form of output heat, not on the surfaces of the housing. A heated housing poses risks to the user who might be injured by touching hot surfaces. It also poses a challenge to make infrared lamps comply with medical standards for these devices.

Alternative solutions in solid state lighting are not attractive due to the high costs involved.

In the current state of the art, natural convection or an electrical fan are typically used to create airflow along or through the device to exhaust the excessive heat generated by the bulb and the filter. As a result, the reflector used to bundle the infrared light as well as the housing get hot during use.

DE 35 09 939 A1 discloses an illumination device which features a reflector housing and multiple fans arranged behind and under the reflector housing. The device further comprises two glass plates covering the reflector housing. The fans direct a part of a circulating air flow between the glass plates and another part into the reflector housing.

### SUMMARY OF THE INVENTION

It is an object of the present invention to provide improved cooling of the outside surface of an illumination device without the necessity of expensive cooler illumination sources or a high constructional effort.

In a first aspect of the present invention an illumination device is presented, the illumination device comprising: a housing having a first side and a second side opposite the first side, at least one inlet arranged at the first side of the housing, at least one outlet arranged at the second side of the housing, an illumination source arranged in the housing, a reflector which at least partially surrounds the illumination source, wherein the reflector has a first reflector side facing the first side of the housing and a second reflector side facing the second side of the housing, a fan arranged on the first side of the housing fluidly connected to the least one inlet, and a plate arranged on the second side of the housing, wherein the at least one outlet is arranged at an outer circumference of the plate, and wherein the fan is configured to draw cold ambient air into the housing through the at least one inlet, guide the cold ambient air from the first reflector side around an outer surface of the reflector through the housing along a predetermined first flow-path arranged in a space between an inner surface of the housing and the outer surface of the reflector and discharge heated air through the at least one outlet.

By way of this arrangement, all components which are directly accessible to a user of the illumination device can be effectively cooled without having to lower radiation power of the illumination source, install complicated cooling elements or use expensive illumination sources with lower heat output. The advantage of the invention is that cold ambient air is sucked into the housing, led along the reflector and thus taking up heat. The hot air is then discharged at the plate which is the hottest outside part of the housing and not circulated through the housing towards a vent at the top or rear of the housing, resulting in heating up the housing or other parts of the device possibly being in contact to the user. The cold ambient air enters the housing at the inlet region and passes the reflector on its outside thereby absorbing heat from its surface. The heated air is discharged at the plate and is prevented from entering the housing again.

A particular advantage is that the reflector is cooled from its back end which is arranged on the first reflector side, whereas the reflector is open to the second reflector side where the plate is arranged. Since the illumination source is arranged closer to the back end of the reflector than to the front end of the reflector, most of the heat is generated at or near the back end of the reflector. Since the reflector is cooled from its back end, it is cooled from its hottest region. The cool air thus hits the hottest region of the reflector first, then surrounds the reflector, and finally the hot air leaves the housing at its second side.

Preferred embodiments of the invention are defined in the dependent claims.

Preferably, the at least one outlet is an annular gap between the plate and the housing. By way of the annular gap which allows heated air to vent around the outer circumference of the plate, a steady discharge of heat can take place, thus cooling the illumination device very efficiently and without any danger to the user of the device.

According to a preferred embodiment of the invention the plate is attached to the housing at positioning points. The positioning points provide each only a small contact area between the plate and the housing. Thus, only a small amount of heat will be transferred from the hot plate to the housing. The housing remains cool to the touch of a user thus decreasing the danger of injuries by burning.

According to another preferred embodiment, the plate is attached to a plate holder which is attached to the housing at positioning points. The plate holder can work as a blind to allow treatment of smaller body parts without loss of heat. Besides, the plate holder can be assembled with the plate to a semi-finished product which can be easily fitted to the housing without damage to one of the components.

Advantageously, interstices are formed between the positioning points. The interstices allow the heated air to leave the housing without heat accumulation in the housing.

Particularly preferable the interstices are part of the annular gap and form the at least one outlet through which the heated air is discharged. Heated air can thus leave around the whole circumference of the plate and not only through one vent opening in the housing.

According to another preferred embodiment, the plate is arranged at a distance to the second reflector side of the reflector. This allows the heated air to travel along further directions through the housing and the reflector and to dissipate more heat from the hot components.

Particularly, a second flow-path advantageously partly extends on the second reflector side of the reflector in a direction parallel to a plane of the plate and directly adjacent to the plate. By way of this, the plate itself which is heated up by the radiation of the illumination source can be cooled. Besides, the air travelling along the second flow path will take up further heat from the second reflector side.

The first and second flow-paths exit the housing through the interstices between the fastening points. This is beneficial for the cooling effect of the whole device since also the air which enters the reflector can be discharged from the housing without heat accumulation in the reflector.

Advantageously the illumination source is a halogen bulb or an incandescent light bulb. Any of these illumination means is widespread, cheap and easy to obtain and can be easily changed even by the user of the device.

According to another embodiment, the reflector extends along a main axis, and the fan is arranged on the main axis. Preferably, both the reflector and the fan are at least substantially symmetrical, preferably exactly symmetrical to said main axis. This allows the best possible cooling of the reflector from all sides. Preferably, the main axis of the reflector extends through the centrum of the plate. It is also preferred that the housing is symmetrical to a mid plane, wherein the main axis of the reflector lies in said mid plane or falls together with said mid plane.

The heated air is preferably discharged from the housing only through the at least one outlet and not through any other outlets, such that a backflow of hot air into the housing is prevented.

According to a further embodiment, the plate includes a glass filter for filtering light of a predetermined wavelength produced by the illumination source. This glass filter is cooled in the above-mentioned way, especially by way of the second second flow-path.

### BRIEF DESCRIPTION OF THE DRAWINGS

These and other aspects of the invention will be apparent from and elucidated with reference to the embodiment(s) described hereinafter. In the following drawings
Fig. 1 shows a sectional view of a preferred embodiment of the invention, and
Fig. 2A-2B shows perspective views of partial sectional views of the embodiment of Fig. 1.

### DETAILED DESCRIPTION OF THE INVENTION

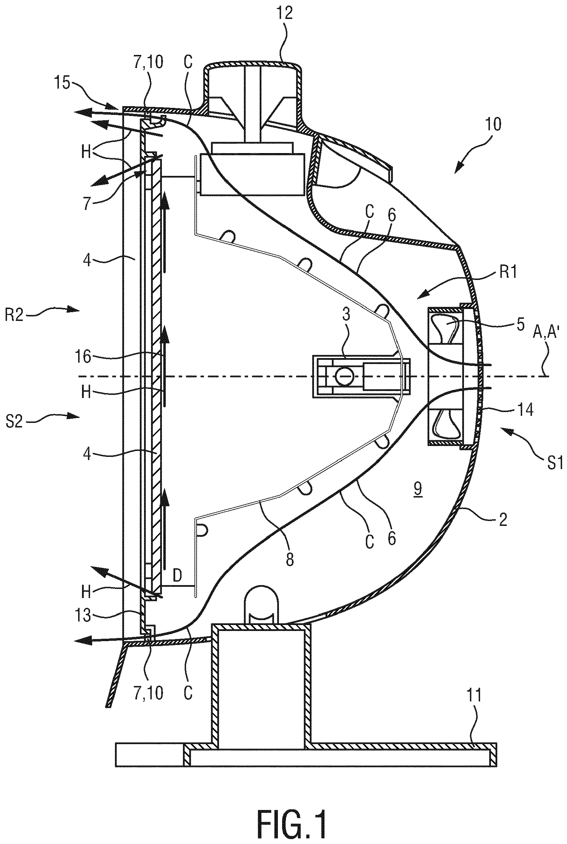

Fig. 1 shows a schematic sectional side view of a first embodiment of an illumination device 1 according to the invention. The illumination device 1 may serve a medical or health care purpose, e.g. for pain relief of blocked muscles and other therapeutic purposes, or for relaxation and leisure. For these purposes, the illumination device 1 will preferably emit radiation in the infrared wavelength range between 0.7 and 1000 µm.

As detailed above, conventional illumination devices often are prone to high temperatures on the outer surfaces with the danger of injury of the user by burning. The reason for heating of the surface is lack of circulation of air and/or cooling means. One possibility of lowering the temperature is use of light sources which are not heating up, but these light sources are expensive.

Thus, the invention proposes a different approach by a specific method of mounting the different components of the illumination device 1 as well as guiding the flow of air in and through the illumination device 1.

The illumination device 1 according to the invention comprises a housing 2, which might for example be shaped in form of a hemisphere or in a parabolic shape or the like. The housing 2 has a first side S1 and a second side S2. In the embodiment of Fig. 1, the first side S1 is the rear side, while the second side S2 is the front side. In the first side S1 at least one inlet 14 is formed. On the second side S2, which is the open side where the radiation of the illumination device 1 is emitted, at least one outlet 15 is formed. The housing 2 can be manufactured from glass, plastics, metal or other suitable materials or mixtures thereof in a generally known fashion. The housing 2 can be mounted on a holder or stand 11 as shown in Fig. 1. An adjustment handle 12 to adjust inclination, direction of emission etc. can be present. The housing 2 further can comprise a power connection (not shown) to be connected to an electric supply.

The illumination device 1 further comprises an illumination source 3, which can be an incandescent light bulb, a halogen bulb or any other suitable light source. Preferably, a halogen bulb is used. The illumination source 3 is arranged in a reflector 8, which itself is arranged in the housing 2. The reflector 8 has a concave shape and a reflecting surface to allow focusing the radiation of the illumination source 3. A first reflector side R1 is orientated towards the first side S1 of the housing 2. A second reflector side R2 is orientated towards the second side S2 of the housing 2. In the embodiment of Fig. 1, the first reflector side R1 represents an outer surface of the reflector 8, whereas the second reflector side R2 represents an inner surface of the reflector 8. The illumination source 3 preferably is arranged in the focal point of the reflector 8 on the second reflector side R2 and on a rotational axis A' of the reflector 8 which preferably extends in approximately the same direction as an axis A of the housing 2. The axes A, A' can also be identical. They extend through the first side S1 and the second side S2. The reflector 8 can be shaped in a generally known fashion from suitable materials.

A transparent plate 4 is arranged on the housing 2. In the embodiment of Fig. 1, the plate 4 covers the second (open) side S2 of the housing 2, thus also covering the second reflector side R2 of the reflector 8. According to Fig. 1, the plate 4 is arranged in a plate holder 13. Alternatively, the plate 4 can be mounted without plate holder directly to the housing 2 as detailed below. The plate 4 is oriented perpendicular to the axis/axes A, A' of the housing 2 and/or of the reflector 8. Alternatively and not further detailed, the plate 4 can be orientated at a different angle with respect to the axes A, A' of the housing 2 and the reflector 8. The plate 4 can be a glass plate or a transparent or translucent plastic plate. The plate 4 can e.g. be transparent to infrared wavelengths only.

The shape of the plate 4 preferably resembles the shape of the second (open) side S2 of the housing 2 and/or of the reflector 8. If the housing 2 and/or the reflector 8 is for example a paraboloid of rotation, the plate 4 will be circular. In the embodiment of Fig. 1, the housing 2 is approximately hemispheric, the reflector 8 is at least partially a paraboloid of rotation, and the plate 4 thus is of circular shape. The plate holder 13 is also ring-shaped in the shown embodiment. Between the plate 4 and the housing 2 an annular gap is provided which forms the at least one outlet 15.

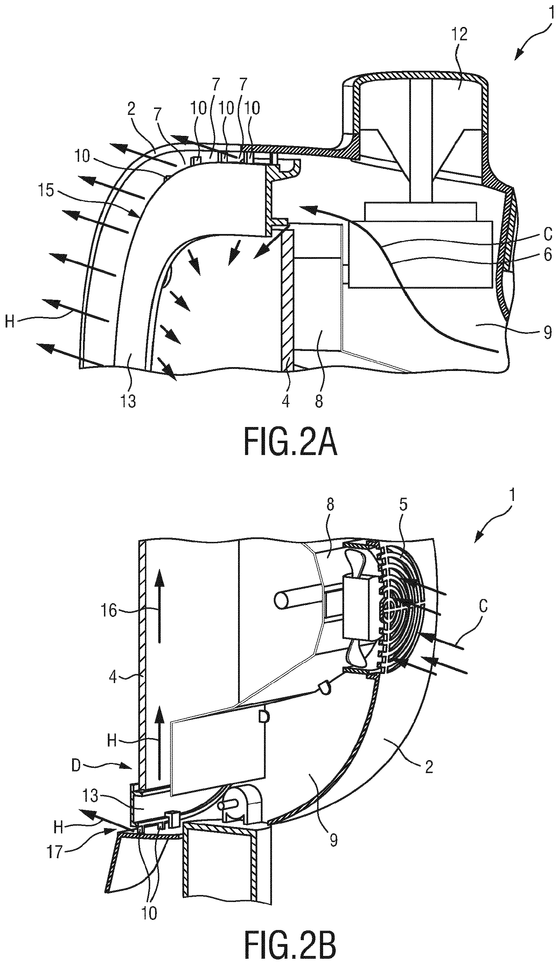

The plate 4 is attached to the housing 2 via the plate holder 13 by positioning points 10 which are spaced apart at predetermined distances. The positioning points 10 are visible in Figs 2A and 2B. By way of securing the plate 4 by a few positioning points 10 only, a thermic decoupling of the plate 4 and the housing 2 and/or of the reflector 8 is possible to a large extent. Heat transfer to the housing 2 can thus be minimized. Alternatively to the plate holder 13 of Fig. 1 the plate 4 can be directly fitted to the housing 2 by way of spacers which then perform the same task as the fastening points 10 of the plate holder 13.

In the housing 2 a fan 5 is arranged, which is located at the first side of the housing 2 and in fluidic connection therewith. Preferably, the fan 5, the illumination source 3 and the plate 4 are arranged on the axes A, A' of the reflector 8 and/or of the housing 2 in the sequence mentioned before. The arrangement of the fan 5 with respect to the illumination source 3 and the plate 4 is crucial for the inventive effect.

As can be seen in Fig. 1, the fan 5 sucks relatively cold ambient air C into the illumination device 1 and directs the cold ambient air C through a space 9 between the housing 2 and the reflector 8 from the first side of the housing S1 towards the first reflector side R1. A first flow-path 6 extends from the inlet 14 to the plate 4 and is guided by the outer shape of the reflector 8 and the inner shape of the housing 2. Between the positioning points 10 of the plate holder 13 or alternatively between the spacers holding the plate 4 directly interstices 7 are formed which connect the space 9 fluidic to the ambient. The entity of interstices 7 forms the annular gap 17 and thus the outlet 15.

The cold ambient air C heats up while travelling through the space 9 and thus transports heat from the reflector 8 in direction of the plate 4. At the plate 4, the cold ambient air C which is now heated up leaves the housing 2 via the interstices 7 as heated air H. Additionally a part of the cold ambient air C is directed to the second reflector side R2 along the plate 4 in a direction parallel to a plane of the plate 4. The direction of flow along this second flow-path 16 can be in an upward direction as shown in Fig. 1, but also a downward or sideward direction would be possible, especially when the cold ambient air C did not completely heat up during transit through the housing 2. Thus not only the housing 2 and the reflector 8, but likewise the plate 4 is cooled, which is also directly accessible by a user of the illumination device 1 and thus should remain as cool as possible. The heated air H travelling along the second flow-path 16 is likewise discharged from the outlet 15.

In Figs 2A and 2B two partial perspective views of the upper part of the illumination device 1 according to Fig. 1 and of the lower part of the illumination device 1 of Fig. 1 are shown.

In Fig. 2A, the plate holder 13 and the interstices 7 between the positioning points 10 are visible. The positioning points 10 in this case are part of the plate holder 13 and are used to position the plate holder 13 to the housing 2. Since the positioning points 10 are spaced apart, only a very small amount of heat can be dissipated from the plate 4 to the housing 2 via the plate holder 13. If the plate 4 is fixed directly to the housing 2, e.g. by spacers (not shown in Figs.), the same effect can be achieved. The less positioning points 10 are present, the less heat will be transferred from the plate 4 to the housing 2. For example, to fix a circular plate 4 to the housing 2, as little as two or three positioning points 10 would be sufficient. The heated air H is vented through the interstices 7 at the circumference of the plate 4. In other words, there will be a flow of heated air H from the interstices 7 as shown by the arrows in Fig. 2A. This flow of heated air H can be used for further purposes within the therapeutic range of purposes of the inventive illumination device 1.

In Fig. 2B, the lower part of the illumination device 1 is shown. The fan 5 is visible, where the cold ambient air C is drawn into the housing 2. After passing the space 9 in the housing 2 along the surface on the first reflector side R1 of the reflector 8, the air has taken up warmth and flows as heated air H through the lower interstices 7 to the outside or partially along the surface of the plate 4 on the second flow-path 16, thus cooling the plate 4. The partially diverted flow-path 16 along the plate 4 extends to further interstices 7 in different locations along the circumference of the plate 4.

By combination of the arrangement of the fan 5 and the respective direction of cold ambient air C in the illumination device 1 with venting the heated air H at or in front of the plate 4 allows an effective transport of heat and an effective cooling of all components which are directly accessible by the user. The heated air H is not circulated back through the illumination device 1 towards a vent in the top or back of the housing like in devices according to the state of the art, resulting in heating up the device from the inside, leading to a hot outer surface. Instead, the cold ambient air C takes up heat from the reflector and leaves the housing 2 without much contact to the inner surface of the housing 2.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Illumination device (1) comprising:
- a housing (2) having a first side (S1) and a second side (S2) opposite the first side (S1),
- at least one inlet (14) arranged at the first side (S1) of the housing (2),
- at least one outlet (15) arranged at the second side (S2) of the housing (2),
- an illumination source (3) arranged in the housing (2),
- a reflector (8) which at least partially surrounds the illumination source (3), wherein the reflector (8) has a first reflector side (R1) facing the first side (S1) of the housing (2) and a second reflector side (R2) facing the second side (S2) of the housing (2),
- a fan (5) arranged on the first side (S1) of the housing (2) and being fluidly connected to the least one inlet (14), and
- a plate (4) arranged on the second side (S2) of the housing (2),
wherein the at least one outlet (15) is arranged at an outer circumference of the plate (4), and
wherein the fan (5) is configured to draw cold ambient air (C) into the housing (2) through the at least one inlet (14), guide the cold ambient air (C) from the first reflector side (R1) around an outer surface of the reflector (8) through the housing (2) along a predetermined first flow-path (6) arranged in a space (9) between an inner surface of the housing (2) and the outer surface of the reflector (8) and discharge heated air (H) through the at least one outlet (15).

2. Illumination device (1) according to claim 1, wherein the at least one outlet (14) is an annular gap (17) between the plate (4) and the housing (2).

3. Illumination device (1) according to claim 1 or 2, wherein the plate (4) is attached to the housing (2) at positioning points (10).

4. Illumination device (1) according to claim 1 or 2, wherein the plate (4) is attached to a plate holder (13) which is attached to the housing (2) at positioning points (10).

5. Illumination device (1) according to claim 3 or 4, wherein interstices (7) are formed between the positioning points (10).

6. Illumination device (1) according to claim 5, wherein the interstices (7) are part of the annular gap (17) and form the at least one outlet (15) through which the heated air (H) is discharged.

7. Illumination device (1) according to one of the preceding claims, wherein the plate (4) is arranged at a distance (D) to the second reflector side (R2) of the reflector (8).

8. Illumination device (1) according to claim 7, wherein a second flow-path (16) partly extends on the second reflector side (R2) of the reflector (8) in a direction parallel to a plane of the plate (4) and directly adjacent to the plate (4).

9. Illumination device (1) according to claim 8, wherein the first and second flow-paths (6, 16) exit the housing (2) through the interstices (7) between the positioning points (10).

10. Illumination device (1) according to one of the preceding claims, wherein the illumination source (3) is a halogen bulb or an incandescent light bulb.

11. Illumination device (1) according to one of the preceding claims, wherein the reflector (8) extends along a main axis, and wherein the fan (5) is arranged on the main axis as well.

12. Illumination device (1) according to claim 11, wherein the main axis of the reflector (8) extends through a central point of the plate (4).

13. Illumination device (1) according to claim 11 or 12, wherein the housing (2) is symmetrical to a mid plane, and wherein the main axis of the reflector (8) lies in said mid plane.

14. Illumination device (1) according to one of the preceding claims, wherein the housing (2) includes only the at least one outlet (15) for discharging the heated air (H).

15. Illumination device (1) according to one of the preceding claims, wherein the plate (4) includes a glass filter for filtering light of a predetermined wavelength produced by the illumination source (3).
